# EUROPÄISCHE PATENTANMELDUNG

(11) **EP 3 025 755 A1**
(43) Veröffentlichungstag der Anmeldung: **01.06.2016**
(21) Anmeldenummer: 15189074.6
(22) Anmeldetag: 09.10.2015
(51) Int. Cl.: A61N 1/365, A61N 1/05, A61N 1/08

(54) **MEDIZINISCHES IMPLANTAT MIT EINER KOMMUNIKATIONSSCHNITTSTELLE**

(30) Priorität: 25.11.2014 US 201462083899 P
(71) Anmelder: BIOTRONIK SE & Co. KG, 12359 Berlin (DE)
(72) Erfinder: Dörr, Thomas, 12437 Berlin (DE); Diebold, Michael, 12169 Berlin (DE)
(74) Vertreter: Galander, Marcus

(57) **Zusammenfassung**

Die Erfindung betrifft ein medizinisches Implantat (100) zum Einführen in den menschlichen und/oder tierischen Körper, umfassend eine Energiequelle (110), einen Pulsgenerator (120), eine Steuereinheit (150) sowie wenigstens ein Elektrodenpaar (130) zur Abgabe von elektrischen Stimulationsimpulsen an ein Körpergewebe. Dabei ist implantatseitig ein Sensor (140) zur Erkennung eines implantatexternen Triggerelements (200) vorgesehen, wobei die Steuereinheit (150) mit dem Sensor (140) gekoppelt und abhängig von einem Sensorsignal des Sensors (140) zum Ausführen einer Anpassungssequenz von Stimulationsparametern aktivierbar ist, in der Prüfstimulationsimpulse ausgebbar sind.

Die Erfindung betrifft ferner ein Verfahren zum Einstellen eines Parametersatzes von therapeutischen elektrischen Stimulationsimpulsen eines Implantats (100).

## Beschreibung

Die Erfindung betrifft ein medizinisches Implantat mit einer Kommunikationsschnittstelle.

Heute bekannte aktive elektronische Implantate zum Einführen in den menschlichen und/oder tierischen Körper besitzen eine bidirektionale Kommunikationsschnittstelle für eine Telemetriefunktion, um Stimulationsparameter einstellen zu können. Die Telemetriefunktion des aktiven elektronischen Implantats benötigt eine für die Telemetrie geeignete Antenne, jeweils angepasst an die zu verwendende Frequenz. Diese Antennengeometrie limitiert dabei jedoch die Möglichkeit zur Miniaturisierung solcher Implantate erheblich.

Der Erfindung liegt die Aufgabe zugrunde, ein Implantat mit Telemetriemöglichkeiten zu schaffen, welches im Vergleich zum Stand der Technik eine weitgehende Miniaturisierung zulässt.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen, der Beschreibung und der Zeichnung.

Es wird ein medizinisches Implantat zum Einführen in den menschlichen und/oder tierischen Körper vorgeschlagen, umfassend eine Energiequelle, einen Pulsgenerator, eine Steuereinheit sowie wenigstens ein Elektrodenpaar zur Abgabe von elektrischen Stimulationsimpulsen an ein Körpergewebe, wobei implantatseitig ein Sensor zur Erkennung eines implantatexternen Triggerelements vorgesehen ist, wobei die Steuereinheit mit dem Sensor gekoppelt und abhängig von einem Sensorsignal des Sensors zum Ausführen einer Anpassungssequenz von Stimulationsparametern aktivierbar ist, in der Prüfstimulationsimpulse ausgebbar sind.

Vorteilhaft kann mittels der Anpassungssequenz eine Bestimmung einer Stimulationsreizschwelle und anschließende Programmierung der Stimulationsenergie durchgeführt werden. Es kann ein miniaturisiertes Implantat ohne Telemetrieantenne realisiert werden, dessen Pulsenergie jedoch patientenindividuell eingestellt werden kann und darüber hinaus ein Indikator für die Erschöpfung der Energiequelle bestimmt werden kann. Stimulationsparameter können insbesondere Amplitude, Pulsbreite und Frequenz umfassen.

Die Erfindung ermöglicht die patientenindividuelle Einstellung von Stimulationsparametern und die Anzeige eines Austauschindikators bei einem Implantat, dessen Kommunikationsschnittstelle bauartbedingt sehr minimal ausgeführt sein kann.

Darüber hinaus kann ein aktives Miniaturimplantat geschaffen werden, das antennenlos ausgebildet ist. Es kann auf Telemetrieeinrichtungen verzichtet werden, so dass zum einen die energetischen Anforderungen reduziert sind und auf geeignete Antennengeometrien für die jeweiligen Telemetrieverfahren verzichtet werden kann. Außerdem kann das Implantat auch in größeren Gewebetiefen, beispielsweise als endokardiales Implantat, eingesetzt werden. In solchen Fällen könnten nur Telemetrieverfahren eingesetzt werden, die eine Kommunikation in großer Gewebetiefe erlauben und entsprechend aufwändig sind.

Gemäß einer günstigen Ausgestaltung kann die Steuereinheit dazu ausgebildet sein, bei Aktivierung durch das Triggerelement eine elektrische Stimulationssequenz mit Prüfstimulationsimpulsen auszulösen. Die Steuereinheit erhält ein Signal des Sensors, welcher auf das Triggerelement reagiert. Vorteilhaft kann die Stimulationssequenz voreingestellt sein.

Gemäß einer günstigen Ausgestaltung kann die Steuereinheit dazu ausgebildet sein, Parameter der Prüfstimulationsimpulse bei der elektrischen Stimulationssequenz zu variieren. Vorteilhaft kann bei einer voreingestellten Stimulationssequenz die Amplitude und/oder die Frequenz der Prüfstimulationsimpulse reduziert werden.

Gemäß einer günstigen Ausgestaltung kann die Steuereinheit dazu ausgebildet sein, bei Erreichen oder Unterschreiten eines unteren Schwellwerts basierend auf einem vorangegangenen Parametersatz der Prüfstimulationsimpulse einen Parametersatz zur Abgabe von therapeutischen elektrischen Stimulationsimpulsen einzustellen. Vorteilhaft kann dabei ein minimal wirksame Wert zuzüglich einer Sicherheitsmarge eingestellt werden. Das Erreichen oder Unterschreiten des unteren Schwellwerts kann durch Hilfsmessungen detektiert werden. So können unter anderem biophysikalische Signale als Reaktion auf die Prüfstimulationsimpulse und/oder physiologische Reaktionen beobachtet werden. Biophysikalische Signale können beispielsweise ein Oberflächen-EKG, ein Elektroenzephalogramm, ein Elektromyogramm sein. Physiologische Reaktionen können beispielsweise Muskelaktivitäten oder Wahrnehmungen des Patienten sein.

Gemäß einer günstigen Ausgestaltung kann die Steuereinheit dazu ausgebildet sein, am Ende der Anpassungssequenz ein Quittungssignal zur Bestätigung des Parametersatz zur Abgabe der therapeutischen elektrischen Stimulationsimpulse zu empfangen und zu verarbeiten. Alternativ kann die Steuereinheit dazu ausgebildet sein, am Ende der Anpassungssequenz eine Sicherheitseinstellung der Stimulationsparameter vorzugeben. Damit ist die Betriebssicherheit des Implantats verbessert.

Gemäß einer günstigen Ausgestaltung können Rechenmittel vorgesehen sein, um aus einem aktuellen Parametersatz zur Abgabe von therapeutischen elektrischen Stimulationsimpulsen und einem Energieinhalt der Energiequelle eine Lebensdauer der Energiequelle abzuschätzen. Die Betriebssicherheit des Implantats wird vorteilhaft verbessert.

Gemäß einer günstigen Ausgestaltung kann die Energiequelle eine Nuklearbatterie umfassen. Die Bestimmung des Zeitpunkts, an dem die Nuklearbatterie erschöpft ist, lässt sich günstigerweise aus der Halbwertszeit der Nuklearbatterie und dem berechneten Stromverbrauch der therapeutischen elektrischen Stimulationsimpulse abschätzen.

Gemäß einer günstigen Ausgestaltung kann das Triggerelement einen Magneten umfassen. Vorteilhaft kann Sensor ein Riesenmagnetowiderstandssensor (GMR-Sensor) sein. GMR-Sensoren reagieren auf ein Magnetfeld, indem ihr elektrischer Widerstand im Magnetfeld stark ansteigt.

Gemäß einer günstigen Ausgestaltung kann das Triggerelement eine galvanische Einkopplung elektrischer Impulse und/oder eine Einkopplung akustischer Signale und/oder eine Einkopplung optischer Signale bewirken. Als mögliche Kombinationen von Triggerelementen und Empfängerelementen im Implantat können beispielsweise eingesetzt werden: Lautsprecher-Mikrofon, Magnet-Reedschalter, gepulster Elektromagnet-GMR-Sensor, Ultraschalltransducer-Ultraschallempfänger, LED-Phototransistor etc. Das Triggerelement kann nach Bedarf ausgewählt werden.

Gemäß einer günstigen Ausgestaltung kann das Implantat als Herzschrittmacher oder Defibrillator oder epikardialer Herzschrittmacher oder elektrodenloser endokardialer Herzschrittmacher oder Neurostimulator oder Muskelstimulator ausgestaltet sein. Vorteilhaft kann ein Implantat geschaffen werden, das nur über eine sehr minimale unidirektionale Kommunikationsschnittstelle verfügt und dennoch eine Stimulationsreizschwellentest nebst Umprogrammierung der Stimulationsparameter und einen Batterieaustauschindikator unterstützt.

Nach einem weiteren Aspekt der Erfindung wird ein System vorgeschlagen mit einem erfindungsgemäßen medizinischen Implantat zum Einführen in den menschlichen und/oder tierischen Körper, zur Abgabe von elektrischen Stimulationsimpulsen an ein Körpergewebe, wobei ein Triggerelement vorgesehen ist, das mit einem implantatseitigen Sensor zusammenwirkt, wobei eine Steuereinheit mit dem Sensor gekoppelt und abhängig von einem Sensorsignal des Sensors zum Ausführen einer Anpassungssequenz von Stimulationsparametern aktivierbar ist, in der Prüfstimulationsimpulse ausgebbar sind.

Das Triggerelement kann einen Magneten umfassen und der Sensor ein Riesenmagnetowiderstandssensor sein, der bei Annäherung des Magneten seinen elektrischen Widerstand ändert. Alternativ oder zusätzlich kann das Triggerelement eine galvanische Einkopplung elektrischer Impulse und/oder eine Einkopplung akustischer Signale und/oder eine Einkopplung optischer Signale bewirken.

Nach einem weiteren Aspekt der Erfindung wird ein Verfahren zum Einstellen eines Parametersatzes von therapeutischen elektrischen Stimulationsimpulsen eines Implantats zum Einführen in den menschlichen oder tierischen Körper vorgeschlagen, bei dem eine Steuereinheit durch ein externes Triggerelement zum Ausführen einer Anpassungssequenz von Stimulationsparametern aktiviert wird, in der Prüfstimulationsimpulse ausgegeben werden. Der Reizschwellentest basiert dabei auf der Auswertung von biophysikalischen Signalen und/oder physiologischen Reaktionen und der Einstellung der letzten als effektiv bewerteten Amplitude plus Sicherheitsmarge. Das Verfahren erlaubt, mittels einer unidirektionale Kommunikation ohne Telemetrieantenne die Pulsenergie eines Implantats patientenindividuell einzustellen, wobei auch ein Indikator für die Batterieerschöpfung bestimmt werden kann.

Gemäß einer günstigen Ausgestaltung können die Prüfstimulationsimpulse solange variiert werden, bis ein unterer Schwellwert erreicht oder unterschritten ist, und dass bei Erreichen oder Unterschreiten des unteren Schwellwerts basierend auf einem vorangegangenen Parametersatz der Prüfstimulationsimpulse ein Parametersatz zur Abgabe von therapeutischen elektrischen Stimulationsimpulsen eingestellt wird.

Gemäß einer günstigen Ausgestaltung kann die Anpassungssequenz durch ein Quittungssignal über das Triggerelement beendet werden oder bei Ausbleiben eines Quittungssignals eine Sicherheitseinstellung aktiviert werden. Die Betriebssicherheit des Implantats kann verbessert werden.

Gemäß einer günstigen Ausgestaltung kann basierend auf einem Parametersatz von therapeutischen elektrischen Stimulationsimpulsen und einem Energieinhalt einer implantatseitigen Energiequelle eine Lebensdauer der Energiequelle abgeschätzt werden. Die Betriebssicherheit des Implantats kann somit verbessert werden.

Die Erfindung ist nachfolgend beispielhaft, anhand von in Zeichnungen dargestellten Ausführungsbeispielen, näher erläutert. Es zeigen in schematischer Darstellung:
- Fig. 1: ein Blockschaltbild eines Implantats mit einem externen Triggerelement;
- Fig. 2: einen Schnitt durch ein endokardiales Implantat nach Figur 1 mit einem externen Triggerelement;
- Fig. 3: einen Ablaufplan einer Anpassungssequenz von Stimulationsimpulsen eines Implantats; und
- Fig. 4: ein Schema zur Bestimmung einer Lebensdauer einer Nuklearbatterie.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typische Ausgestaltungen der Erfindung wieder und sollen die Erfindung nicht auf die dargestellten Ausgestaltungen beschränken.

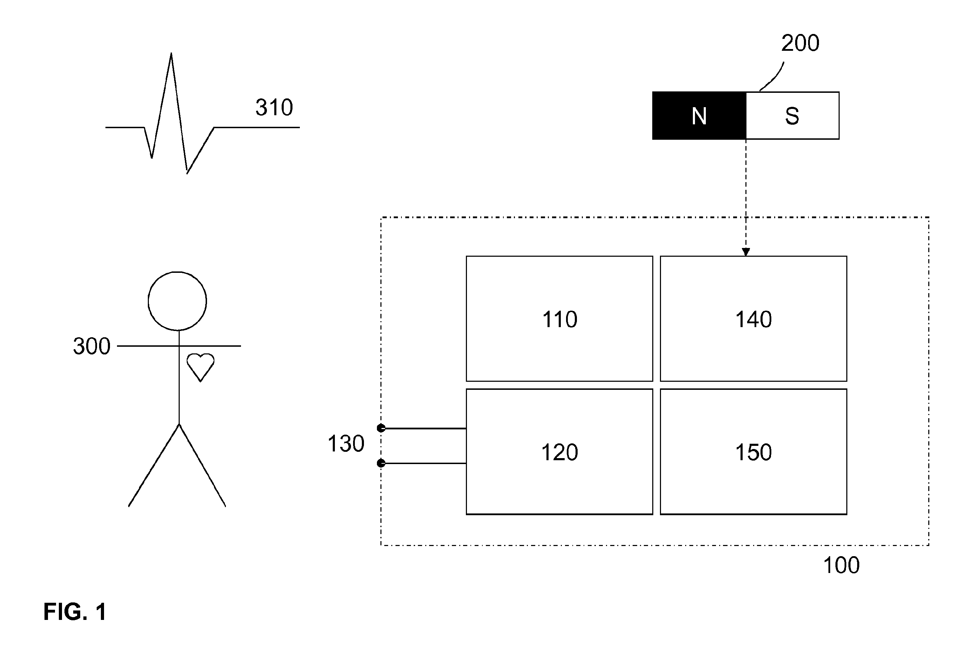

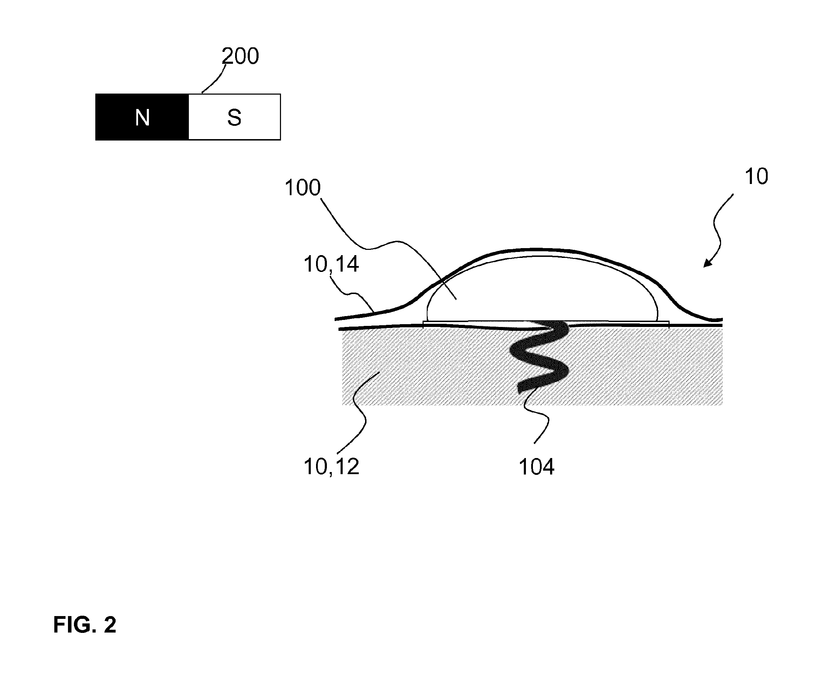

Figur 1 zeigt zur Veranschaulichung der Erfindung eine Anordnung mit einem Blockschaltbild eines medizinischen Implantats 100 zum Einführen in den menschlichen und/oder tierischen Körper, mit einem externen Triggerelement 200 und einem Patienten 300. Das Implantat 100 ist dazu ausgebildet, therapeutische Stimulationsimpulse an das Gewebe abzugeben. Figur 2 zeigt in Schnittansicht ein derartiges medizinisches Implantat 100 als endokardiales Miniaturimplantat, das an einem Implantationsort 10 eingesetzt ist, im Falle des endokardialen Implantats 100 zwischen Myokard 12 und Perikard 14. Das Implantat 100 ist im Myokard 12 mit einer Fixierhelix 110 befestigt. Ein solches Implantat 100 erfordert eine Minimierung seiner geometrischen Abmessungen und seines Gewichts. Vorteilhaft kann bei dem Miniaturimplantat auf Telemetrieeinrichtungen verzichtet werden, so dass zum einen die energetischen Anforderungen reduziert sind und auf geeignete Antennengeometrien für die jeweiligen Telemetrieverfahren verzichtet werden kann. Bei einem endokardialen Implantat können nur Telemetrieverfahren eingesetzt werden, die eine Kommunikation in großer Gewebetiefe erlauben.

Das medizinische Implantat 100 umfasst, wie in Figur 1 dargestellt ist, eine Energiequelle 110, einen Pulsgenerator 120, einen Sensor 140 sowie eine Steuereinheit 150 und wenigstens ein Elektrodenpaar 130. Die Energiequelle kann beispielsweise eine Nuklearbatterie sein.

Die Steuereinheit 150 steuert den Pulsgenerator zur Abgabe von elektrischen Pulsen an, die im Normalbetrieb therapeutische Stimulationsimpulse sind, welche über das als Dipol ausgebildetes Elektrodenpaar 130 an das Myokardgewebe (Figur 2) eines Patienten 300 abgegeben werden.

Zum Einstellen von patientenindividuellen Stimulationsparametern kann die Steuereinheit 150, abhängig von einem Sensorsignal des Sensors 140, zum Ausführen einer Anpassungssequenz von Stimulationsparametern aktiviert werden, in der Prüfstimulationsimpulse ausgegeben werden.

Der Sensor 140 kann das Vorhandensein eines einfachen Triggerelements 200 über dem Patienten 300 erkennen und der Steuereinheit 150 ein entsprechendes Sensorsignal weiterleiten. Im gezeigten Beispiel ist das Triggerelement 200 ein starker Permanentmagnet und der Sensor 140 ein GMR-Sensor. Zusätzlich wird im gezeigten Beispiel ein Oberflächen-EKG 310 des Patienten 300 abgeleitet, um damit die Effektivität der Stimulation erkennen zu können.

Die Steuereinheit 150 ist dazu ausgebildet, bei Aktivierung durch das Triggerelement 200 am Pulsgenerator 120 eine elektrische Stimulationssequenz mit Prüfstimulationsimpulsen auszulösen. Es wird eine Anpassungssequenz der Stimulationsparameter ausgeführt, bei der eine Reizschwelle detektiert werden soll, ab der Stimulationsimpulse wirksam sind. Die Wirksamkeit der Prüfstimulationsimpulse kann mit dem Oberflächen-EKG 310 erkannt werden. Stimulationsparameter können Amplitude und/oder Pulsbreite und/oder Frequenz der Stimulationsimpulse sein.

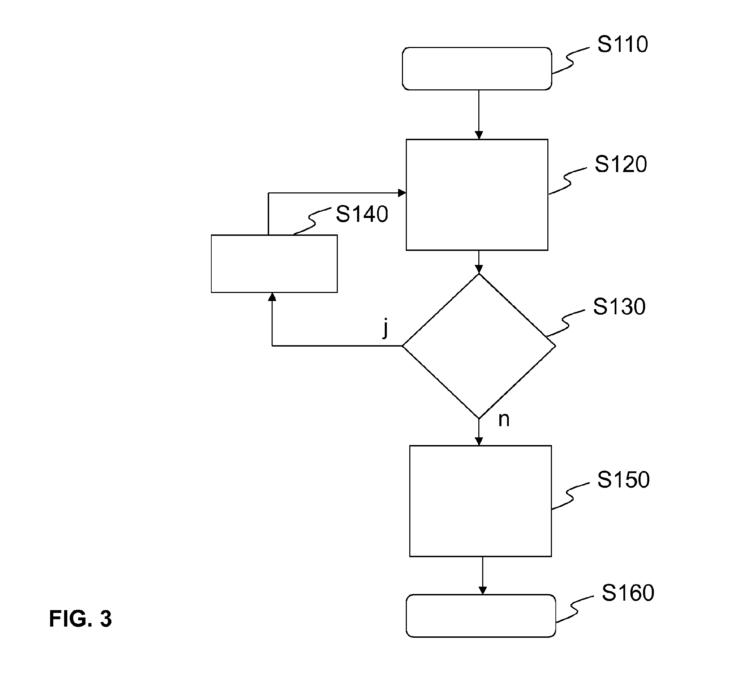

Der Ablauf der Anpassungssequenz zur Detektion einer patientenindividuellen Reizschwelle mit einer Amplitudenanpassung der Prüfstimulationsimpulse ist anhand eines Ablaufplans in Figur 3 erläutert.

Die Anpassungssequenz kann durch eine definierte einleitende Startsequenz in Schritt S110 beginnen. Eine Startsequenz kann beispielsweise ein Auflegen und Wegnehmen des Magneten in der Nähe des Implantats 100 sein, was vom Sensor 140 an die Steuereinheit 150 weitergemeldet wird (Figur 1). Dann wird der Magnet wieder aufgelegt. Die Anpassungssequenz wird solange durchgeführt, wie der Magnet aufgelegt ist, d.h. solange das Triggerelement 200 nach der Startsequenz vom Sensor 140 erkannt wird (Figur 1). Während diese Anpassungssequenz läuft, werden in Schritt S120 wiederholt eine bekannte Anzahl von Prüfstimulationsimpulsen mit definierter Amplitude und Pulsbreite abgegeben und in Schritt S130 geprüft, ob der Magnet noch aufgelegt ist. Ist dies der Fall ("j" in Schritt S130 im Ablaufschema), zeigt dies der Steuereinheit 150 im Implantat 100 an, dass der Anwender die aktuelle Stimulation als effektiv bewertet, so dass für den folgenden Teil der Anpassungssequenz die Stimulationsenergie in vorgegebener Weise reduziert wird. Dies wird wiederholt, bis der Magnet abgehoben wird und der Sensor 140 der Steuereinheit 150 anzeigt, dass die Stimulation ineffektiv war ("n" in Schritt S130 im Ablaufschema). In diesem Fall ist die Reizschwelle erreicht, und die Steuereinheit 150 beendet die Anpassungssequenz. Die Steuereinheit 150 stellt für die permanente therapeutische Stimulation die zuletzt als gültig bewertete Stimulationsenergie plus eine vordefinierte Sicherheitsmarge in Schritt S150 ein.

Durch eine optionale Magnet-Sequenz in Schritt S160 kann der Anwender diese Einstellung mit einem Quittungssignal quittieren und damit in der Steuereinheit 150 bestätigen, andernfalls wird eine Sicherheitseinstellung aktiviert.

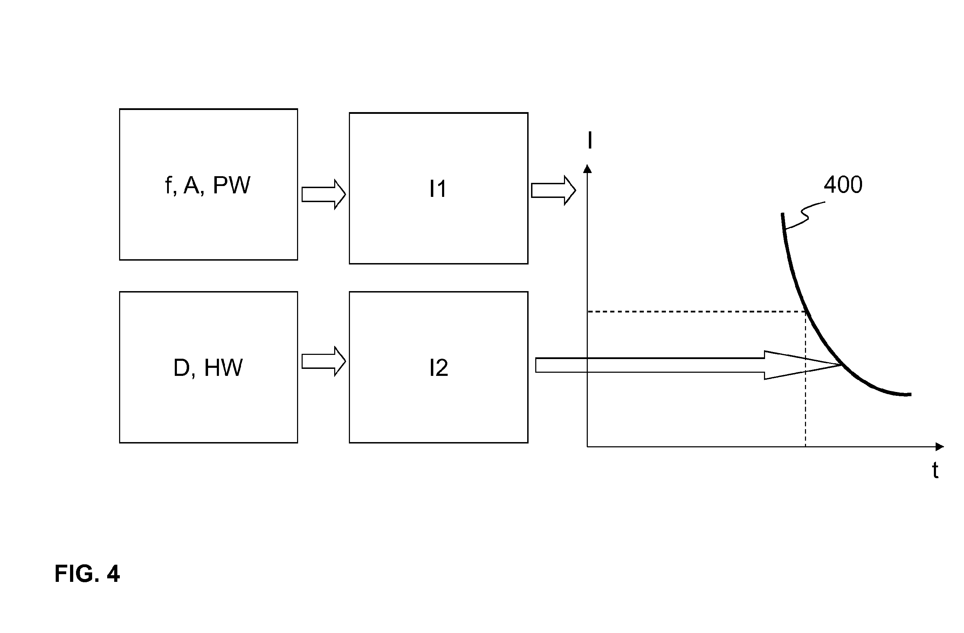

In Figur 4 ist ein Schema zur Bestimmung einer Lebensdauer einer Nuklearbatterie, die als Energiequelle 110 (Figur 1) eingesetzt werden kann, gezeigt. Die Steuereinheit 150 stellt dazu die Information über die ausgewählte Stimmulationsamplitude bereit, in dem die Auswahl der Stimulationsamplitude nach einem festen Schema ausgewählt wird. Mittels eines externen EKG Schreibers werden die Signale dann aufgezeichnet und die Stimulationsamplituden können ausgezählt werden. In Kombination mit dem Batteriestartdatum und/oder dem Produktionsdatum der Energiequelle (110) (z.B. eine Nuklearbatterie) kann dann aus einer Tabelle (z.B. Bestandteil des Manuals oder auf einer Web-Page) die verbleibende Restlaufzeit unter Annahme der aktuellen Stimulationsamplitude herausgelesen werden.

Die Bewertung der verbleibenden Betriebszeit bei Verwendung der Nuklearbatterie beruht auf den bekannten und mittels der Anpassungssequenz in Figur 3 eingestellten Stimulationsparametern Frequenz f, Amplitude A und Pulsbreite PW, die in einen maximalen Stromverbrauch I1 des Implantats umgerechnet werden. Da eine Nuklearbatterie unabhängig vom entnommenen Strom immer einen Maximalstrom liefern kann, der einzig von der Halbwertszeit HW des eingesetzten Radionuklids abhängig ist (Kurve 400), kann aus dieser Halbwertszeit HW und dem Alter D der Nuklearbatterie ein maximal lieferbarer Strom I2 über die Zeit berechnet werden. Anhand dieser Daten kann die verbleibende Betriebszeit abgelesen werden. Die Steuereinheit 150 kann bei Annäherung der Betriebsdauer des Implantats 100 an die maximale Lebensdauer ein entsprechendes Indikatorsignal abgeben, beispielsweise bei einer Neueinstellung von Stimulationsparametern.

Die Erfindung ermöglicht es, einen sehr kleinen implantierbaren Pulsgenerator mit Reizschwelleneinstellung und Austauschindikator zu realisieren, ohne dass dafür eine Telemetriefunktion implementiert werden muss.

## Patentansprüche

1. Medizinisches Implantat (100) zum Einführen in den menschlichen und/oder tierischen Körper, umfassend eine Energiequelle (110), einen Pulsgenerator (120), eine Steuereinheit (150) sowie wenigstens ein Elektrodenpaar (130) zur Abgabe von elektrischen Stimulationsimpulsen an ein Körpergewebe, **dadurch gekennzeichnet, dass** implantatseitig ein Sensor (140) zur Erkennung eines implantatexternen Triggerelements (200) vorgesehen ist, wobei die Steuereinheit (150) mit dem Sensor (140) gekoppelt und abhängig von einem Sensorsignal des Sensors (140) zum Ausführen einer Anpassungssequenz von Stimulationsparametern aktivierbar ist, in der Prüfstimulationsimpulse ausgebbar sind.

2. Implantat nach Anspruch 1, **dadurch gekennzeichnet, dass** die Steuereinheit (150) dazu ausgebildet ist, bei Aktivierung durch das Triggerelement (200) eine elektrische Stimulationssequenz mit Prüfstimulationsimpulsen auszulösen.

3. Implantat nach Anspruch 1 oder 2, **dadurch gekennzeichnet, dass** die Steuereinheit (150) dazu ausgebildet ist, Parameter der Prüfstimulationsimpulse bei der elektrischen Stimulationssequenz zu variieren.

4. Implantat nach einem der vorhergehenden Ansprüche, **dadurch gekennzeichnet, dass** die Steuereinheit (150) dazu ausgebildet ist, bei Erreichen oder Unterschreiten eines unteren Schwellwerts basierend auf einem vorangegangenen Parametersatz der Prüfstimulationsimpulse einen Parametersatz zur Abgabe von therapeutischen elektrischen Stimulationsimpulsen einzustellen.

5. Implantat nach Anspruch 4, **dadurch gekennzeichnet, dass** die Steuereinheit (150) dazu ausgebildet ist, am Ende der Anpassungssequenz ein Quittungssignal zur Bestätigung des Parametersatz zur Abgabe der therapeutischen elektrischen Stimulationsimpulse zu empfangen und zu verarbeiten.

6. Implantat nach Anspruch 4, **dadurch gekennzeichnet, dass** die Steuereinheit (150) dazu ausgebildet ist, am Ende der Anpassungssequenz eine Sicherheitseinstellung der Stimulationsparameter vorzugeben.

7. Implantat nach einem der vorhergehenden Ansprüche, **dadurch gekennzeichnet, dass** Rechenmittel vorgesehen sind, um aus einem aktuellen Parametersatz zur Abgabe von therapeutischen elektrischen Stimulationsimpulsen und einem Energieinhalt der Energiequelle (110) eine Lebensdauer der Energiequelle (110) abzuschätzen.

8. Implantat nach einem der vorhergehenden Ansprüche, **dadurch gekennzeichnet, dass** die Energiequelle (110) eine Nuklearbatterie umfasst.

9. Implantat nach einem der vorhergehenden Ansprüche, **gekennzeichnet durch** eine Ausgestaltung als Herzschrittmacher oder Defibrillator oder epikardialem Herzschrittmacher oder elektrodenlosem endokardialem Herzschrittmacher oder Neurostimulator oder Muskelstimulator.

10. System mit einem medizinischen Implantat (100) zum Einführen in den menschlichen und/oder tierischen Körper, zur Abgabe von elektrischen Stimulationsimpulsen an ein Körpergewebe, nach einem der vorhergehenden Ansprüche, wobei ein Triggerelement (200) vorgesehen ist, das mit einem implantatseitigen Sensor (140) zusammenwirkt, wobei eine Steuereinheit (150) mit dem Sensor (140) gekoppelt und abhängig von einem Sensorsignal des Sensors (140) zum Ausführen einer Anpassungssequenz von Stimulationsparametern aktivierbar ist, in der Prüfstimulationsimpulse ausgebbar sind.

11. System nach Anspruch 10, **dadurch gekennzeichnet, dass** das Triggerelement (200) einen Magneten umfasst und der Sensor (140) ein Riesenmagnetowiderstandssensor ist.

12. System nach Anspruch 10 oder 11, **dadurch gekennzeichnet, dass** das Triggerelement (200) eine galvanische Einkopplung elektrischer Impulse und/oder eine Einkopplung akustischer Signale und/oder eine Einkopplung optischer Signale bewirkt.

13. Verfahren zum Einstellen eines Parametersatzes von therapeutischen elektrischen Stimulationsimpulsen eines Implantats (100) zum Einführen in den menschlichen oder tierischen Körper, insbesondere nach einem der Ansprüche 1 bis 9, **dadurch gekennzeichnet, dass** eine Steuereinheit (150) durch ein externes Triggerelement (200) zum Ausführen einer Anpassungssequenz von Stimulationsparametern aktiviert wird, in der Prüfstimulationsimpulse ausgegeben werden.

14. Verfahren nach Anspruch 13, **dadurch gekennzeichnet, dass** die Prüfstimulationsimpulse solange variiert werden, bis ein unterer Schwellwert erreicht oder unterschritten ist, und dass bei Erreichen oder Unterschreiten des unteren Schwellwerts basierend auf einem vorangegangenen Parametersatz der Prüfstimulationsimpulse ein Parametersatz zur Abgabe von therapeutischen elektrischen Stimulationsimpulsen eingestellt wird.

15. Verfahren nach Anspruch 13 oder 14, **dadurch gekennzeichnet, dass** die Anpassungssequenz durch ein Quittungssignal über das Triggerelement (200) beendet wird oder dass bei Ausbleiben eines Quittungssignals eine Sicherheitseinstellung aktiviert wird.

16. Verfahren nach einem der Ansprüche 13 bis 15, **dadurch gekennzeichnet, dass** basierend auf einem Parametersatz von therapeutischen elektrischen Stimulationsimpulsen und einem Energieinhalt einer implantatseitigen Energiequelle (110) eine Lebensdauer der Energiequelle (110) abgeschätzt wird.
